# EUROPEAN PATENT APPLICATION

(11) **EP 2 457 566 A1**
(43) Date of publication of application: **30.05.2012**
(21) Application number: 10802077.7
(22) Date of filing: 21.07.2010
(51) Int. Cl.: A61K 31/192, A61K 31/196, A61K 31/405, A61K 31/5415, A61K 31/616, A61K 45/00, A61K 45/06, A61K 47/26, A61K 47/48, A61P 17/02, A61P 29/00, A61P 43/00

(54) **EXTERNAL PREPARATION CONTAINING NSAIDs AND METHOD FOR PRODUCING THE EXTERNAL PREPARATION**

(30) Priority: 24.07.2009 JP 2009173609
(71) Applicant: Next 21 K.K., Tokyo 113-0033 (JP); The University of Tokyo, Tokyo 113-8654 (JP)
(72) Inventor: TEI, Yuichi, Tokyo 113-8654 (JP); SASAKI, Nobuo, Tokyo 113-8654 (JP); SUZUKI, Shigeki, Tokyo 113-0033 (JP)
(74) Representative: Moore, Joanne Camilla
(86) International application number: PCT/JP2010/004669
(87) International publication number: WO 2011/010456

(57) **Abstract**

Disclosed is an external preparation containing nonsteroidal anti-inflammatory drugs (NSAIDs), which is suppressed in cytotoxicity induced by the NSAIDs. Also disclosed is a method for producing the external preparation. The present invention is based on the finding that skin disorders induced by nonsteroidal anti-inflammatory drugs (NSAIDs) can be suppressed when the NSAIDs form intermolecular compounds together with trehalose, which is an example of disaccharides. A disaccharide other than trehalose may be used therefor.

## Description

### Technical Field

The present invention relates to an external preparation containing an intermolecular compound formed from a nonsteroidal anti-inflammatory drug (NSAIDs) and a disaccharide, and a method for producing the external preparation, and the like.

### Background Art

NSAIDs have been used widely for external preparations having anti-inflammatory effects. The NSAIDs, however, have a side-effect to induce cell dysfunction. For that reason, the external preparations containing the NSAIDs have a defect in which skin disorders are evoked.

Japanese Patent Application JP-A No. 8-208459 (Patent Document 1) discloses tapes for transdermal administration mixing trehalose and a trehalose derivative. The Patent Document describes NSAIDs as examples of drugs contained in the tapes. When the tape contains NSAIDs, skin disorders may possibly be induced by the NSAIDs. It should be noted that, in the Patent Document described above, trehalose is used only for preventing rash caused by a base of the tape.

### Prior Art Documents

### Patent Documents

Patent Document 1: JP-A No. 8-208459

### Disclosure of the Invention

### Problems to be Solved by the Invention

The present invention aims at providing external preparations capable of effectively suppressing skin disorders induce by NSAIDs.

### Means for Solving Problems

The present invention is based on the finding that the skin disorders induced by NSAIDs can be effectively suppressed when intermolecular compounds are formed from a disaccharide (such as trehalose) and NSAID.

A first aspect of the present invention is an external preparation containing an intermolecular compound obtained from a nonsteroidal anti-inflammatory drug (NSAIDs) and a disaccharide and being capable of suppressing the skin disorders induced by NSAIDs by the intermolecular compound. As shown in Examples described below, when NSAIDs and disaccharide are formed into an intermolecular compound, the skin disorders induced by the NSAIDs can be effectively suppressed. The external preparations of the invention are, accordingly, preferably used as external preparations having an effect of effectively suppressing the skin disorders induced by NSAIDs. Certainly, the external preparations of the present invention have also anti-inflammatory effects caused by the NSAIDs.

In a preferable first aspect of the invention, the intermolecular compound is obtained by drying a mixed solution containing the NSAIDs and the disaccharide. As shown in Examples described below, the intermolecular compound can be obtained by drying a mixed solution in which both the NSAIDs and the trehalose are dissolved. As the thus obtained intermolecular compound is contained, the skin cell disorder induced by the NSAIDs can be effectively suppressed. Here, at least one compound selected from trehalose, maltose, sucrose and lactose can be used as the disaccharide.

In a preferable embodiment of the first aspect of the present invention, the disaccharide is trehalose, and the intermolecular compound contains the NSAIDs and the trehalose in a weight ratio of 10:1 to 1:50. As shown in Examples described below, when the weight ratio is within the range described above, the intermolecular compound is formed from the NSAIDs and the trehalose. The external preparation having such an intermolecular compound can effectively suppress the skin disorder induced by the NSAIDs.

In a preferable embodiment in the first aspect of the present invention, the NSAIDs is an acidic NSAIDs. The NSAIDs used in the present invention more preferably contains any one or two or more of indomethacin, ibuprofen, aspirin, diclofenac sodium, mefenamic acid, piroxicam, felbinac, loxoprofen, ketoprofen, flurbiprofen, glycol salicylate, glycyrrhetinic acid, Loxonin, suprofen, bufexamac, ufenamate, 5-aminosalicylic acid and naproxen as the acidic NSAIDs. As shown in Examples described below, when an external preparation containing the intermolecular compound formed from the acidic NSAIDs and the trehalose is used, the skin disorder induced by the NSAIDs can be effectively suppressed.

In a preferable embodiment of the first aspect of the present invention, the external preparation further includes an oleaginous base. That is, this embodiment relates to a hydrophobic ointment containing the intermolecular compound of the NSAIDs and the disaccharide. As demonstrated in Examples described below, the effect of suppressing the cell dysfunction induced by the NSAIDs is significantly increased when the hydrophobic ointment is contained. Thus, the external preparation of the present invention contains preferably the oleaginous base.

A preferable embodiment in the first aspect of the present invention further contains a cosolvent. As described below, the cosolvent stabilizes the intermolecular compound of the NSAIDs and the disaccharide. Accordingly, when the external preparation further includes the cosolvent, the external preparation containing the intermolecular compound described above can be preferably used as an external preparation having an effect of suppressing the skin disorder induced by the NSAIDs.

In a preferable embodiment in the first aspect of the present invention, the external preparation further contains a local anesthetic. Examples of the local anesthetic contain one kind or two kinds or more of lidocaine, tetracaine, procaine, dibucaine, benzocaine, bupivacaine, mepivacaine, ethyl aminobenzoate, diethylaminoethyl para-butylaminobenzoate, meprylcaine, oxypolyethoxydodecane, and scopolia extract, a salt thereof.

In a preferable embodiment in the first aspect of the present invention, the NSAIDs is indomethacin. In this embodiment, tops of a first peak and a second peak on a DSC curve of the intermolecular compound, obtained by measurement using a differential scanning calorimetry (DSC), preferably appear at 80 to 95°C and 260 to 270°C, respectively.

In a preferable embodiment in the first aspect of the present invention, the NSAIDs is ibuprofen. In this embodiment, tops of a third peak and a fourth peak on a DSC curve of the intermolecular compound, obtained by measurement using a differential scanning calorimetry (DSC), preferably appear at 175 to 190°C and 130 to 145°C, respectively. The first peak and the second peak of the intermolecular compound preferably appear in a range of 95 to 105°C and a range of 70 to 80°C, respectively. In addition, the DSC curve of the intermolecular compound preferably does not appear in a range of 110 to 130°C or a range of 200 to 210°C

In a preferable embodiment in the first aspect of the present invention, the NSAIDs is aspirin. In this embodiment, tops of a first peak and a second peak on a DSC curve of the intermolecular compound, obtained by measurement using a differential scanning calorimetry (DSC), preferably appear at 110 to 120°C and 135 to 145°C, respectively.

In a preferable embodiment in the first aspect of the present invention, the NSAIDs is diclofenac sodium. In this embodiment, tops of a first peak and a second peak on a DSC curve of the intermolecular compound, obtained by measurement using a differential scanning calorimetry (DSC), preferably appear at 90 to 100°C and 130 to 145°C, respectively.

In a preferable embodiment in the first aspect of the present invention, the NSAIDs is mefenamic acid. In this embodiment, tops of a first peak and a second peak on a DSC curve of the intermolecular compound, obtained by measurement using a differential scanning calorimetry (DSC), appear at 225 to 235°C and 90 to 110°C, respectively. In addition, in the intermolecular compound, peaks also appear at 180 to 190°C and 250 to 265°C. Further, the absolute values (values measured by DSC) of the tops of the first peak and the second peak are preferably higher than absolute values of tops of peaks appearing at 90 to 110°C and 225 to 235°C on a DSC curve of the mefenamic acid, obtained by measurement using DSC.

In a preferable embodiment in the first aspect of the present invention, the NSAIDs is piroxicam. In this embodiment, tops of a first peak and a second peak on a DSC curve of the intermolecular compound, obtained by measurement using a differential scanning calorimetry (DSC), appear at 90 to 105°C and 195 to 205°C, respectively. The second peak appearing at 195 to 205°C is a main peak (the peak having the strongest intensity in that range) in a range of 190 to 220°C. In addition, the absolute values of the tops of the first peak and the second peak are preferably lower than absolute values of tops of peaks appearing at 90 to 105°C and at 195 to 205°C on a DSC curve of the piroxicam, obtained by measurement using DSC.

In a preferable embodiment in the first aspect of the present invention, the NSAIDs is 5-aminosalicylic acid. In this embodiment, the intermolecular compound has a DSC curve, obtained by measurement using a differential scanning calorimetry (DSC), in which a peak appears at 207 to 215°C but it does not appear at 95 to 105°C.

In a preferable embodiment in the first aspect of the present invention, the NSAIDs is ketoprofen. In this embodiment, the intermolecular compound has a DSC curve, obtained by measurement using a differential scanning calorimetry (DSC), in which peaks appear at 90 to 95°C and 230 to 235°C, but a peak does not appear at 180 to 220°C.

In a preferable embodiment in the first aspect of the present invention, the NSAIDs is naproxen. In this embodiment, the intermolecular compound has a DSC curve, obtained by measurement using a differential scanning calorimetry (DSC), in which peaks appear at 90 to 95°C and 234 to 237°C, but a peak does not appear at 225 to 233°C.

A second aspect of the present invention relates to a method for producing an external preparation including the steps of: preparing a mixed solution containing a nonsteroidal anti-inflammatory drug (NSAIDs) and trehalose; and drying the mixed solution. The step of drying the mixed solution includes a step of mixing the dried, mixed solution with a base. As shown in Examples described below, when such a production method is employed, the NSAIDs is intermolecularly reacted with the trehalose to form an intermolecular compound, and external preparations capable of suppressing the skin disorders induced by NSAIDs can be produced.

### Effect of the Invention

As the external preparation of the present invention contains the intermolecular compound of the disaccharide and the NSAIDs, NSAIDs-containing external preparations capable of suppressing the skin disorders induced by NSAIDs can be provided.

### Brief Description of Drawings

FIG. 1 shows charts, replacing for a drawing, which show DSC results of trehalose alone, indomethacin alone, a mixture of indomethacin and trehalose and a lyophilized mixture of the indomethacin and trehalose.
FIG. 2 shows charts, replacing for a drawing, which show DSC results of trehalose alone, ibuprofen alone, a mixture of ibuprofen and trehalose, and a lyophilized mixture of ibuprofen and trehalose.
FIG. 3 shows charts, replacing for a drawing, which show DSC results of trehalose alone, aspirin alone, a mixture of aspirin and trehalose, and a lyophilized mixture of aspirin and trehalose.
FIG. 4 shows charts, replacing for a drawing, which show DSC results of trehalose alone, diclofenac alone, a mixture of diclofenac sodium and trehalose, and lyophilized mixture of diclofenac sodium and trehalose.
FIG. 5 shows charts, replacing for a drawing, which show DSC results of trehalose alone, piroxicam alone, a mixture of piroxicam and trehalose, and lyophilized mixture of piroxicam and trehalose.
FIG. 6 shows charts, replacing for a drawing, which show DSC results of trehalose alone, mefenamic acid alone, a mixture of mefenamic acid and trehalose, and a lyophilized mixture of mefenamic acid and trehalose.
FIG. 7 shows graphs, replacing for a drawing, which show that an ointment preparation containing an intermolecular compound of NSAIDs and trehalose suppresses a cell dysfunction induced by the NSAIDs.
FIG. 8 shows graphs, replacing for a drawing, which show cytotoxicity test results in control experiment in Example 3 of the present invention.
FIG. 9 shows graphs, replacing for a drawing, which show cytotoxicity test results in Example 3 in which indomethacin is used as an NSAIDs to be contained in an external preparation.
FIG. 10 shows graphs, replacing for a drawing, which show cytotoxicity test results in Example 3 in which diclofenac is used as an NSAIDs to be contained in an external preparation.
FIG. 11 shows graphs, replacing for a drawing, which show cytotoxicity test results in Example 3 in which ibuprofen is used as an NSAIDs to be contained in an external preparation.
FIG. 12 shows graphs, replacing for a drawing, which show cytotoxicity test results in Example 3 in which piroxicam is used as an NSAIDs to be contained in an external preparation.
FIG. 13 shows graphs, replacing for a drawing, which show cytotoxicity test results in Example 3 in which felbinac is used as an NSAIDs to be contained in an external preparation.
FIG. 14 shows graphs, replacing for a drawing, which show cytotoxicity test results in Example 4 in which an intermolecular compound with an NSAIDs is formed using maltose, together with other results.
FIG. 15 shows charts, replacing for a drawing, which show DSC results of trehalose alone, 5-ASA alone, a mixture of 5-ASA and trehalose, and lyophilized mixture of 5-ASA and trehalose.
FIG. 16 shows charts, replacing for a drawing, which show DSC results of trehalose alone, ketoprofen alone, a mixture of ketoprofen and trehalose, and a lyophilized mixture of ketoprofen and trehalose.
FIG. 17 shows charts, replacing for a drawing, which show DSC results of trehalose alone, naproxen alone, a mixture of naproxen and trehalose, and lyophilized mixture of naproxen and trehalose.
FIG. 18 shows FT-IR spectra of aspirin alone, trehalose, a mixture of aspirin and trehalose, and lyophilized mixture of aspirin and trehalose.
FIG. 19 shows FT-IR spectra of 5-ASA alone, trehalose, a mixture of 5-ASA and trehalose, and lyophilized mixture of 5-ASA and trehalose.
FIG. 20 shows FT-IR spectra of diclofenac alone, trehalose, a mixture of diclofenac and trehalose, and lyophilized mixture of diclofenac and trehalose, wherein FIG. 20 (a) shows spectra in a range of 650 to 4000 cm⁻¹, and FIG. 20(b) shows partially enlarged spectra 20 of FIG. 20 (a).
FIG. 21 shows FT-IR spectra of indomethacin alone, trehalose, a mixture of indomethacin and trehalose, and lyophilized mixture of indomethacin and trehalose, wherein FIG. 21 (a) shows spectra in a range of 650 to 4000cm, and FIG. 21(b) shows partially enlarged spectra 21 of FIG. 21 (a).
FIG. 22 shows FT-IR spectra of ibuprofen alone, trehalose, a mixture of ibuprofen and trehalose, and a lyophilized mixture of ibuprofen and trehalose, wherein FIG. 22 (a) shows spectra in a range of 650 to 4000 cm⁻¹, and FIG. 22(b) shows partially enlarged spectra 22 of FIG. 22 (a).
FIG. 23 shows FT-IR spectra of ketoprofen alone, trehalose, a mixture of ketoprofen and trehalosem, and lyophilized mixture of ketoprofen and trehalose, wherein FIG. 23 (a) shows spectra in a range of 650 to 40000 cm⁻¹, and FIG. 23 (b) shows partially enlarged spectra 23 of FIG. 23 (a).
FIG. 24 shows FT-IR spectra of naproxen alone, trehalose, a mixture of naproxen and trehalose, and a lyophilized mixture of naproxen and trehalose, wherein FIG. 24 (a) shows spectra in a range of 650 to 4000 cm⁻¹, and FIG. 24 (b) shows partially enlarged spectra 24 of FIG. 24 (a).
FIG. 25 shows FT-IR spectra of piroxicam alone, trehalose, a mixture of piroxicam and trehalose, and a lyophilized mixture of piroxicam and trehalose, wherein FIG. 25 (a) shows spectra in a range of 650 to 4000 cm⁻¹, and FIG. 25 (b) shows partially enlarged spectra 25 of FIG. 25 (a).
FIG. 26 shows FT-IR spectra of mefenamic acid alone, trehalose, a mixture of mefenamic acid and trehalose, and a lyophilized mixture of mefenamic acid and trehalose, wherein FIG. 26 (a) shows spectra in a range of 650 to 4000 cm⁻¹, and FIG. 26 (b) shows partially enlarged spectra 26 of FIG. 26 (a).

### Description of Embodiments

The first aspect of the present invention relates to external preparations containing an intermolecular compound (the compound according to the present invention) of a nonsteroidal anti-inflammatory drug (NSAIDs) and trehalose, and thereby capable of suppressing the skin disorders induced by the NSAIDs. That is, the external preparations of the invention can exhibit anti-inflammatory effects of the NSAIDs, and can suppress the skin disorders induced by the NSAIDs.

In the present invention, NSAIDs is not particularly limited, and known NSAIDs may be used. Examples of the NSAIDs may include acidic NSAIDs and basic NSAIDs. The acidic NSAIDs include carboxylic acid NSAIDs and enol acid NSAIDs. Examples of the carboxylic acid NSAIDs may include salicylic acid NSAIDs such as aspirin and sodium salicylate NSAIDs; arylacetic acid NSAIDs such as indomethacin and etodolac NSAIDs; propionic acid NSAIDs such as ibuprofen, naproxen, ketoprofen and loxoprofen NSAIDs; fenamic acid NSAIDs such as mefenamic acid and tolfenamic acid NASIDs; and phenylacetic acid NSAIDs such as diclofenac sodium and felbinac NSAIDs. Examples of the enol acid NSAIDs may include pyrazolone NSAIDs such as ketophenylbutazone, and clofezone; and oxicam NSAIDs such as piroxicam, lornoxicam, tenoxicam, meloxicam and ampiroxicam. Examples of the basic NSAIDs may include epirizole, tiaramide, and emorfazone. As the examples of the NSAIDs, NSAIDs other than the acidic NSAIDs and the basic NSAIDs, that is, which belong to another category, can be used. The NSAIDs belonging to the other category can be exemplified by dimethylisopropylazulene. In the external preparation of the present invention, any of the acidic NSAIDs, the basic NSAIDs and the NSAIDs belonging to the other category may be used. In the present invention, the acidic NSAIDs is preferably used. As shown in Examples described below, the external preparations of the invention can effectively suppress the cell dysfunctions, because they contain the acidic NSAIDs. In the present invention, the intermolecular compound may contain one kind or two kinds or more of NSAIDs. The acidic NSAIDs preferably contains one or two or more of indomethacin, ibuprofen, aspirin, diclofenac sodium, mefenamic acid, piroxicam, felbinac, loxoprofen, ketoprofen, flurbiprogen, glycol salicylate, glycurrhetinic acid, Loxonin, suprofen, bufexamac, and ufenamate. When 2 kinds or more of the NSAIDs is contained, they may belong to the same category (for example, the NSAIDs is the salicylic acid NSAIDs), or they may belong to different categories (for example, the NSAIDs is the salicylic acid NSAIDs and the arylacetic acid NSAIDs). These NSAIDs may be produced according to a known method, or commercially available one may be appropriately used.

The NSAIDs may be a simple compound, a salt of the compound, or a solvate such as a hydrate of the compound.

The trehalose used in the present invention is a disaccharide in which two molecules of D-glucose are bonded to each other. The trehalose has three kinds of isomers which have different binding mode from each other: an α, α-form (α-D-glucopyranosyl=α-D-glucopyranoside); an α, β-form (β-D-glucopyranosyl=α-D-glucopyranoside); and α β, β-form (β-D-glucopyranosyl=β-D-glucopyranoside). In the present invention, the production method, the purity and the properties of the trehalose are not restricted so long as one or more of these isomers are contained in an effective amount. Commercially available trehalose can be appropriately utilized.

In the specification, the intermolecular compound of the NSAIDs and the trehalose may contain another substance, in addition to the compound formed from the NSAIDs and the trehalose. Further, the intermolecular compound of the NSAIDs and the trehalose may be in the state of a salt, a hydrate or a solvate. Examples of the method for distinguishing the intermolecular compound from the mixtures of the NSAIDs and the trehalose may include a DSC (differential scanning calorimetry) method, an FTIR (Fourier transform infrared spectroscopy) method, an XPS (X-ray photoelectron spectrometry) method, and an NMR (nuclear magnetic resonance) method. The term "intermolecular compound" refers to a compound in which two or more substances are bonded to each other by intermolecular interaction. The term "intermolecular interaction" refers to an interaction in which two or more molecules act with each other through a bonding power between the molecules. Examples of the intermolecular interaction may include an ionic bond, complex binding, hydrophobic bond, hydrogen bond, and von der Waals binding. The phenomenon in which the NSAIDs and the trehalose are formed into the intermolecular compound can be examined using a known method.

In the present invention, the amount of the NSAIDs contained in the external preparation may be exemplified by from 0.01 to 10 parts by weight based on 100 parts by weight of the whole amount of the external preparation. Those skilled in the art may arbitrarily determine the amount of the NSAIDs to be contained in the external preparation depending on the kind of the NSAIDs used, the use of the external preparation, and the dosage form of the external preparation. The amount of the trehalose contained in the external preparation may be exemplified by from 0.001 to 50 parts by weight based on 100 parts by weight of the whole amount of the external preparation.

In the external preparation of the invention, a mixed ratio of the NSAIDs and the trehalose is exemplified by from 10:1 to 1:50. When the ratio of the NSAIDs is too high, it is not preferable that the intermolecular compound is formed insufficiently. On the other hand, when the ratio of the trehalose is too high, it is not preferable that the pharmacological effects of the NSAIDs become weak. The mixed ratio of the NSAIDs and the trehalose, therefore, is preferably from 5:1 to 1:45, more preferably from 2:1 to 1:40, further more preferably from 1:1 to 1:30.

In a preferable embodiment in the first aspect of the present invention, the external preparation of the invention further contains a cosolvent. In the present invention, the amount of the cosolvent contained in the external preparation may be from 1 to 50 parts by weight based on 100 parts by weight of the whole amount of the external preparation. The cosolvent used in the present invention is a solvent having both hydrophilic property and oleaginous property. Many of the NSAIDs used in the present invention have the oleaginous property. On the other hand, the trehalose has the hydrophilic property. As described above, the NSAIDs and the trehalose have the different properties from each other, and in the external preparation containing the NSAIDs and the trehalose, therefore, the intermolecular interaction, which occurs between the NSAIDs and the trehalose in the external preparation, may sometimes disappear during or after the drug formation. As a result, the NSAIDs and the trehalose do not form the intermolecular compound, but the two compounds existing separately. As described above, the intermolecular compound of the NSAIDs and the trehalose in the external preparation of the invention cannot be always stable during and after the drug formation. When the cosolvent having high affinity with both the NSAIDs and the trehalose is contained in the external preparation, however, the intermolecular interaction between the NSAIDs and the trehalose can be kept stably. When the external preparation of the invention contains the cosolvent, accordingly, the intermolecular compound of the NSAIDs and the trehalose through the intermolecular interaction can exist stably during and after the drug formulation. When the external preparation of the invention contains the cosolvent, accordingly, the effect of suppressing the skin disorder induced by the NSAIDs can be effectively obtained by the trehalose.

In the external preparation of the present invention, examples of the cosolvent may include alcohol solvents, ether solvents, glycerol, propylene glycol, and mixtures thereof. Examples of the alcohol solvent may include methanol, ethanol, isopropanol, liquid phenol, and benzyl alcohol. Examples of the ether solvent may include tetrahydrofuran and dioxane. In the present invention, as the cosolvent, the alcohol solvents are preferable, and ethanol and liquid phenol are more preferable. The alcohol solvents have high cell membrane permeability. When the external preparation of the invention contains the alcohol solvent having the high cell membrane permeability, the cell membrane permeability of the intermolecular compound of the NSAIDs and the trehalose is also increased. As described above, when the external preparation of the invention contains the alcohol solvent as the cosolvent, the cell membrane permeability of the NSAIDs is increased, whereby the pharmacological effects of the NSAIDs can be increased inside the cells. Of the alcohol solvents, ethanol and liquid phenol have small cell dysfunction. When the ethanol or the liquid phenol is used as the cosolvent in the external preparation of the invention, accordingly, the pharmacological effects caused by the NSAIDs can be effectively obtained while the cell dysfunction induced by the cosolvent can be suppressed.

In a preferable embodiment in the first aspect of the present invention, the mixed solution containing the NSAIDs and the trehalose is dried, and the thus obtained product is used as the intermolecular compound of the NSAIDs and the trehalose. As shown in Examples described below, when the external preparation contain the intermolecular compound obtained by dissolving both the NSAIDs and the trehalose and drying the solution, the resulting external preparation can preferably suppress the cell dysfunction.

The external preparation of the invention may contain a base for external preparation. Examples of the base for external preparation may include oleaginous bases (hydrophobic bases), hydrophilic bases, and suspensible bases.

Examples of the oleaginous base used in the invention may include fatty acid esters, aromatic carboxylic acid esters, phosphoric acid esters, higher fatty acid triglycerides, surfactants, terpenes, vaseline, liquid paraffin, plastibases, silicon, natural rubber, synthetic rubber, resins, lanoline, beeswax, white beeswax, cacao butter, laurin butter, simple ointments, and witepsol. The oleaginous bases may be used alone or as a mixture of two kinds or more. The intermolecular compound in the invention may be directly mixed with the oleaginous base, or may be uniformly dispersed in the oleaginous base using a solubilizer.

In the present invention, the fatty acid ester used as the oleaginous base has an alcohol component of a monohydric or polyhydric alcohol, and a fatty acid component of a monovalent or polyvalent fatty acid, wherein the alcohol and fatty acid may have an unsaturated bond. Examples of the fatty acid ester may include methyl stearate, stearyl stearate, sorbitan monostearate, polyoxyethylene sorbitan tristearate, stearic acid monoglyceride, palmitic acid monoglyceride, oleic acid monoglyceride, and dioctyl sebacate. Examples of the aromatic carboxylic acid ester may include distearyl phtalate, dioctyl phthalate, didecyl phthalate, dicyclohexyl phthalate, diphenyl phthalate, and dibehenyl phthalate. Examples of the phosphoric acid ester may include lauryl phosphate, stearyl phosphate, trioleyl phosphate, and tridecyl phosphate. Examples of the higher fatty acid triglyceride may include vegetable fats and oils, and animal fats and oils. Examples of the vegetable fat and oil may include jojoba oil, olive oil, castor oil, peppermint oil, and safflower oil. Examples of the animal fat and oil may include beef tallow fatty acid triglyceride, lard, and squalane.

Examples of the hydrophilic base used in the present invention may include hydrophilic ointment, vanishing cream, hydrophilic petrolatum, purified lanolin, absorptive ointment, hydrous lanolin, hydrophilic plastibase, cold cream, macrogols (polyethylene glycol) ointment, glycerin, and liquid paraffin. Examples of the suspensible base may include fat-free ointment, and FAG base.

The external preparation of the invention may further contain a pharmaceutically acceptable carrier or medium. Examples of the pharmaceutically acceptable carrier or medium contained in the external preparation of the invention may include a stabilizer, an anti-oxidant, a preservative, an emulsifier, and a base. Examples of the stabilizer may include albumin, gelatin, sorbitol, mannitol, lactose, sucrose, maltose and glucose. Examples of the anti-oxidant may include sodium sulfite, ascorbic acid, tocophenol, cysteine hydrochloride, thioglycolic acid, and catechol. Examples of the preservative may include phenolic substances, benzoic acid, sorbic acid, borax, thimerosal, and benzalkonium chloride. Examples of the emulsifier may include calcium oleate, sodium lauryl sulfate, polysorbate, gum arabic, sodium alginate, pectin, and senega saponin. Here, those skilled in the art can arbitrarily select the pharmaceutically acceptable carrier or medium to be contained in the external preparation of the invention from known pharmaceutically acceptable carriers or mediums, and can utilize them.

The external preparation of the invention may further contain a local anesthetic. The "local anesthetic" is not particularly limited so long as it has conventionally been used as a local anesthetic for medical use. Examples of the local anesthetic may include lidocaine, tetracaine, procaine, dibucaine, benzocaine, bupivacaine, mepivacaine, ethyl aminobenzoate, diethylaminoethyl para-butylaminobenzoate, meprylcaine, oxypolyethoxydodecane, scopolia extract, and salts thereof. It is preferably to select and to use one kind or two kinds or more thereof. Of these local anesthetics, lidocaine, tetracaine, procaine, dibucaine, benzocaine, bupivacaine, and mepivacaine, or salts thereof are preferable, and lidocaine is particularly preferable. The salts of the compound forming the local anesthetic may be exemplified by hydrochlorides, carbonates, or sulfates.

The "local anesthetic" used has preferably a positive ion group such as an amino group or a carbonyl group. This is because it would appear that when the positive ion group is ionically bonded to the carboxyl group of the NSAIDs, each ion group is covered with a hydrophobic part to improve the pharmacokinetics, whereby the irritancy to skins can be ameliorated. For example, ethyl aminobenzoate has a carbonyl group and a primary amino group; tetracaine has a carbonyl group, and primary and tertiary amino groups; procaine has a carbonyl group, and primary and tertiary amino groups; lidocaine has a carbonyl group, and secondary and tertiary amino groups; mepivacaine has a carbonyl group, and secondary and tertiary amino groups; and bupivacaine has a carbonyl group, and secondary and tertiary amino groups. It would appear that the interaction thereof with the NSAIDs ameliorates the skin irritation.

In the present invention, the blending ratio of the local anesthetic to the intermolecular compound is not particularly limited, and the ratio of the local anesthetic is preferably 0.1 to 1.5 parts by weight per part by weight of the intermolecular compound used in the invention. Similarly, the molar ratio of the both is not particularly limited, and the local anesthetic is preferably blended so that the molar ratio thereof is 0.1 to 1.8 relative to the intermolecular compound used in the invention.

The second aspect of the present invention relates to a method for producing an external preparation capable of suppressing the skin disorders induced by NSAIDs, in which an intermolecular compound is formed by an intermolecular interaction of a nonsteroidal anti-inflammatory drug (NSAIDs) and trehalose. The production method of the present invention includes a step in which a mixed solution containing NSAIDs and trehalose is prepared (step 1); a step in which the mixed solution is dried (step 2); and a step in which the intermolecular compound, which has been dried in the drying step, is mixed with a base (step 3). When an external preparation is produced, substances to be contained in the external preparation are usually added to and mixed with a base for external preparation as they are. According to such a usual method, however, an intermolecular compound cannot be formed from the NSAIDs and the trehalose. The external preparation produced in the usual method, accordingly, may possibly induce cell dysfunctions by the NSAIDs. On the other hand, the production method of the invention intentionally includes a step in which the NSAIDs is dissolved together with the trehalose to prepare the mixed solution, and the resulting mixed solution is dried. As shown in Examples described below, the intermolecular compound formed according to the production method of the invention is one in which the trehalose forms the intermolecular compound together with the NSAIDs. When the trehalose forms the intermolecular compound together with the NSAIDs, the cell dysfunction by the NSAIDs can be suppressed. When the production method of the invention is employed, accordingly, external preparations capable of effectively suppressing cell dysfunctions induced by NSAIDs can be produced.

In the present invention, the step of preparing the mixed solution (step 1) is a step in which a mixed solution wherein the NSAIDs is dissolved together with the trehalose is prepared. Any known solution used for a drug product such as water, distilled water, ion-exchanged water, MiliQ water or physiological saline may be used as the solution for dissolving the NSAIDs and the trehalose. The mixed solution may be prepared by mixing a trehalose solution with an NSAIDs solution, which have been previously dissolved separately, by mixing and dissolving one of the trehalose and NSAIDs in the state of a powder into a solution dissolving the other, or by adding powdery trehalose and powdery NSAIDs to a solution and dissolving them. In order to dissolve the low-soluble NSAIDs, the NSAIDs may be once dissolved in ethanol or the like, and then it may be mixed with a solution for dissolving it together with trehalose. In this step, the amount of the solution dissolving both the NSAIDs and the trehalose is not particularly limited so long as the NSAIDs and the trehalose are dissolved therein. The amount of the solution dissolving the both is specifically from one to 100 times the whole amount of the NSAIDs and the trehalose. In this step, the NSAIDs and the trehalose can be mixed by means of stir mixing or shake mixing. The stirring speed may be 0.5 revolutions per minute to 100 revolutions per minute upon stir mixing. The shaking speed may be 5 to 200 times per minute upon shake mixing. Those skilled in the art can arbitrarily set the stirring speed or the shaking speed depending on the amounts of the NSAIDs, the trehalose and the solution dissolving the both. The temperature at which the mixed solution is prepared in this step is not particularly limited so long as the NSAIDs and the trehalose are dissolved. The temperature is specifically from 5 to 50°C.

In the present invention, the drying step (step 2) is a step in which the mixed solution wherein the both are dissolved is dried to obtain an intermolecular compound. Examples of the drying step in the invention may include a lyophilizing step, fluidized bed granulation and drying step, a spray drying step, and drying, pulverizing and granulation step.

### [Lyophilizing Step]

In the present invention, the lyophilizing step is a step wherein water is sublimed from a frozen sample performed under reduced pressure. The lyophilizing step is performed as follows: (1) A sample (a mixed solution) is allowed to stand at an ambient temperature of 4°C under an ordinary pressure for 2 to 3 hours to cool the sample (cooling step). (2) The sample is allowed to stand at an ambient temperature of -50°C under an ordinary pressure for 12 to 15 hours to freeze the sample (freezing step). (3) The sample is allowed to stand at an ambient temperature of -20°C under an ordinary pressure for 4 to 6 hours to crystallize the sample (crystallization step). (4) The sample is allowed to stand at an ambient temperature of -50°C under an ordinary pressure for 14 to 16 hours to re-freeze the sample (re-freezing step). (5) The sample is allowed to stand at an ambient temperature of -13°C under a pressure of 10 to 20 kPa (under high vacuum) for 24 to 26 hours (a first drying step). (6) The sample is allowed to stand at an ambient temperature of 24°C under a pressure of 10 to 20 kPa (under high vacuum) for 10 to 121 hours (a second drying step). (7) The sample is allowed to stand at an ambient temperature of 24°C under an ordinary pressure. As described above, according to the lyophilizing method, the sample is frozen at a low temperature, and water (ice) is sublimed under high vacuum to remove it. The lyophilized product in the present invention can be produced by the method described above. The method is not limited to the steps described above, and those skilled in the art can appropriately change the parameters of each step such as the temperature, the pressure and the time.

### [Fluidized Bed Granulation and Drying Step]

In the present invention, the fluidized bed granulation and drying step is a step wherein the sample containing water is flown while warm air is applied to the sample, whereby the sample is granulated and dried. The fluidized bed granulation and drying step is performed using a known fluidized bed granulating and drying machine according to the following steps: (1) A warm air having a temperature of 50 to 100°C is applied to a sample (a mixed solution) at a wind speed of 1 to 2 m/second for 10 to 30 minutes while the sample is stirred (rough drying step). (2) A warm air having a temperature of 20 to 50°C is applied to the sample at a wind speed of 2 to 3 m/second for 30 minutes to one hour (granulation step). (3) A warm air having a temperature of 50 to 100°C is applied to the sample at a wind speed of 1 to 2 m/second for 30 minutes to 2 hours (drying step). (4) A cool air having a temperature of 5 to 20°C is applied to the sample at a wind speed of 1 to 2 m/second for 10 to 60 minutes (cooling step). As described above, in the fluidized bed granulation and drying step, the warm air is applied to the sample and the sample is flown in the air to dry the sample, thereby granulating the sample. The intermolecular compound in the present invention can be produced by the steps described above. The method, however, is not limited to the steps described above, and those skilled in the art can appropriately change the parameters of each step such as the temperature and the wind speed according to the amount of water in the sample.

### [Spray Drying Step]

In the present invention, the spray drying step is a step wherein a sample solution is sprayed from a nozzle having a small pore size together with a hot air to minute liquid droplets in a chamber, whereby the sample is dried for a short time. The spray drying step is performed using a known spray dryer according to the following steps: (1) A sample (mixed solution) is sprayed from a nozzle having a pore size of 0.5 to 1 mm together with a hot air having a temperature of 100 to 300°C under an air pressure of 0.5 to 2.5 kg/m² at a flow rate of 25 to 50 L/minute into a chamber (spraying step). (2) A hot air having a temperature of 150 to 300°C is applied to the sprayed sample at a speed of 0.5 to 1 m/second to dry the sample for 30 seconds to 5 minutes (drying step). As described above, according to the spray drying step, the hot air is applied to the minute liquid droplets, which are produced by spraying the sample in the high temperature chamber, whereby the intermolecular compound is formed into granules. The intermolecular compound in the invention can be produced by the steps described above. In the invention, however, the method is not limited to the steps described above, and those skilled in the art can appropriately change the parameters of each step such as the temperature and the time.

### [Drying, Pulverizing and Granulation Step]

In the present invention, the drying, pulverizing and granulation step is a step wherein the sample containing water is dried, and then it is pulverized to obtain granules. The drying, pulverizing and granulation step is performed according to the following steps: (1) A sample (a mixed solution) is stirred at a stirring speed of 10 to 100 revolutions per minute for 1 to 5 hours, while a warm air having a temperature of 50 to 80°C is applied to the sample (drying step). (2) A cool air having a temperature of 5 to 15°C is applied to the dried sample to cool the sample (cooling step). (3) The cooled sample is pulverized using a pulverizer (pulverizing step). (4) The pulverized samples are put through a sieve having a pre-determined size (sieving step). As described above, according to the drying, pulverizing and granulation step, the sample is once formed into large blocks, and then the blocks are pulverized into particles having a desired size. The intermolecular compound in the invention can be produced by the steps described above. The method, however, is not limited to the steps described above, and those skilled in the art can appropriately change the parameters of each step such as the temperature and the time.

In the drying step in the present invention, the obtained intermolecular compound has preferably a water content (% by mass) of 0.01 to 50%. When the water content of the intermolecular compound is too high, the intermolecular compound is easily separated from the base after the drug formulation. For that reason, the water content of the intermolecular compound in the present invention is preferably 30% or less, more preferably 20% or less, further more preferably 10% or less.

In the present invention, the mixing step (step 3) is a step wherein the intermolecular compound obtained from the drying step is mixed with a base. According to the mixing step (step 3), first the base is heated to a temperature higher than the melting point of the base to dissolve it. After the dissolution, the intermolecular compound is added to the base, and the mixture is stirred until it is uniformly mixed. The intermolecular compound may be added to the base which has been once dissolved and then has been cooled to a temperature around its melting point. In the mixing step in the invention, the base and the intermolecular compound may be stirred using a known stirrer such as a kneader. When the stirring speed is too fast, the intermolecular interaction of the intermolecular compound is broken. When the rotation speed is too low, the time until the base and the intermolecular compound are mixed uniformly is too long. For that reason, the stirring speed in the mixing step in the invention is exemplified by from 1 to 100 revolutions per minute, preferably from 2 to 50 revolutions per minute, more preferably from 5 to 20 revolutions per minute. In the mixing step in the production method of the invention, when the size of the intermolecular compound to be added to the base is too large, it is difficult to uniformly mix it with the base.

The mixing ratio of the intermolecular compound and the base used in the production method of the invention is, for example, from 2:1 1 to 1:100. Those skilled in the art can appropriately change the mixing ratio of the intermolecular compound and the base depending on the kind and amount of the NSAIDs contained in the dried intermolecular compound. According to the production method of the invention, a pharmaceutically acceptable carrier or medium may be added to the intermolecular compound and the base. Examples of the pharmaceutically acceptable carrier or medium used in the production method of the invention may include a stabilizer, an anti-oxidant, a preservative and an emulsifier. Examples of the stabilizer may include albumin, gelatin, sorbitol, mannitol, lactose, sucrose, maltose, and glucose. Examples of the anti-oxidant may include sodium sulfite, ascorbic acid, tocophenol, cysteine hydrochloride, thioglycolic acid, and catechol. Examples of the preservative may include phenolic substances, benzoic acid, sorbic acid, borax, thimerosal, and benzalkonium chloride. Examples of the emulsifier may include calcium oleate, sodium lauryl sulfate, polysorbate, gum arabic, sodium alginate, pectin, and senega saponin. Those skilled in the art can appropriately select the pharmaceutically acceptable carrier or medium, and add them in the appropriate amount in the preparation steps of the invention.

According to the method for producing the external preparation of the invention, the trehalose forms the intermolecular compound together with the NSAIDs, and therefore the external preparation capable of effectively suppressing the cell dysfunction induced by the NSAIDs can be produced. Those skilled in the art can appropriately change the parameters of each step depending on the kind and characteristics of the NSAIDs, and the dosage form and the use of the external preparation.

In the step of preparing the mixed solution (step 1) in the invention, a cosolvent may be contained in the mixed solution. Further, according to the present invention, in the step of mixing the intermolecular compound with the base (step 3), a cosolvent may be contained, in addition to the intermolecular compound and the base.

When the cosolvent is added in the step of preparing the mixed solution (step 1), the amount of the cosolvent may be from 1 to 50 parts by weight based on 100 parts by weight of the whole amount of the mixed solution containing the cosolvent.

In the step of preparing the mixed solution (step 1) in the invention, a cosolvent may be contained in the mixed solution. When the cosolvent is added in the step of mixing the intermolecular compound with the base (step 3) in the invention, the amount of the cosolvent added may be from 0.1 to 10 parts by weight based on 100 parts by weight of the whole amount of the external preparation. In the present invention, a case in which the cosolvent still remains in the external preparation after the drug formulation is preferable, because the intermolecular compound of the NSAIDs and the trehalose is stabilized. When the cosolvent is added to the mixed solution in step 1, a part of the cosolvent is evaporated in the drying step (step 2). For that reason, the addition of the cosolvent is preferably performed in the step of mixing the intermolecular compound with the base (step 3).

Examples of the dosage form of the external preparation of the invention may include oil ointments, hydrophilic ointments, suppositories, cataplasms, sprays, gels, lotions, eye drops, and creams. Those skilled in the art can appropriately produce an external preparation having a desired dosage form according to the use using a known method.

The cataplasms, which are the external preparations, often use a polyhydric alcohol such as glycerol and propylene glycol, a natural polymer such as gelatin, a synthetic polymer such as carboxyvinyl polymer, a tackifier such as polybutene, an excipient such as kaolin, preservative, a gelling agent and the like as the base component. In this invention, those known components may be used, and, if necessary, effective components other than the intermolecular compound may be added.

The method for producing the external preparation of the invention has no particular limitations. For example, after the intermolecular compound of the invention and the lipophilic amine are previously dissolved in an oil solvent, the oil solution may be added to a base in the external preparation, thereby producing the external preparation, or the intermolecular compound of the invention, the lipophilic amine and the oil solvent may be separately poured into the base, and then the intermolecular compound in the invention is dissolved in the oil solvent together with the lipophilic amine, whereby the external preparation may be produced.

In the case of the external preparation having an ointment dosage form, an oil solvent which liquefies when it is heated but solidifies at room temperature to have an appropriate viscosity is used, and the intermolecular compound in the invention and the lipophilic amine are added to and dissolved in the oil solvent while they are warmed, whereby the external preparation may be produced. According to this method, the external preparation can be produced without using other base components.

The suppository can be produced using, for example, a method disclosed in JP-A No. 2000-212065. In this case, a base and a drug used in the suppository are not particularly limited. For example, the suppository may be produced with consideration for the safety and the like, using hard fat, cacao butter, glycerogelatin, hydrogenated vegetable oil, a mixture of polyethylene glycols having a molecular weight different from another, or polyethylene glycol fatty acid ester. The suppository can be produced, for example, by melting the suppository base, uniformly dispersing or dissolving the drug and other necessary components in the molten base, putting the mixture in a mold of the suppository under a certain temperature condition, and solidifying the mixture at around room temperature.

In a case of an external liquid agent, from emulsion type agents to transparent solubilized agents can be prepared depending on the surfactant used. In a case of an aerosol agent, from scattering type agents to mousse can be produced by appropriately selecting the components forming the base.

In case of the ointment, an oil ointment can be formed by kneading an oil solution in which the intermolecular compound in the invention and the lipophilic amine are dissolved in, for example, another oil base. When an appropriate amount of water is added and a surfactant is skillfully used, an O/W type or W/O type ointment can be prepared. If the suppository is considered to be as an extension of the ointment, the hardness of the base may be controlled to be slightly hard, and the melting point may be controlled so that the suppository is molten at a body temperature.

In a case of a patch, for example, a water-containing cataplasm can be prepared by kneading the above-stated oil solution in which the intermolecular compound in the invention is dissolved with a base for water-containing cataplasm. When it is kneaded with a rubber or plastic base, a plaster agent or the like can be prepared.

The external preparation of the invention containing the intermolecular compound formed by the intermolecular interaction of the NSAIDs and the trehalose has the anti-inflammatory action, the analgesic action and antipyretic action of the NSAIDs. The external preparation of the invention containing the intermolecular compound formed by the intermolecular interaction of the NSAIDs and the trehalose can be preferably utilized in a treatment or prevention method in which the external preparation of the invention is administered in an effective amount to a patient whose disease is effectively treated or prevented by the NSAIDs, because the cell dysfunction induced by the NSAIDs can be suppressed. That is, the present invention also provides a treatment method or a prevention method of administering the external preparation containing the intermolecular compound of the NSAIDs and the trehalose to a subject.

In addition, the present invention provides a use of the intermolecular compound of the NSAIDs and the trehalose for producing the external preparation capable of suppressing the skin disorder induced by the NSAIDs. In this use, all of the embodiments described above can be combined and used.

The explanations described above are made taking the trehalose as the example of the compound forming the intermolecular compound with the NSAIDs. In the present invention, however, the intermolecular compound may be formed using maltose, sucrose or lactose, instead of trehalose with the NSAIDs. In addition, the intermolecular compound may be formed using two or more disaccharides selected from the group consisting of trehalose, maltose, sucrose and lactose, with the NSAIDs. The compound forming the intermolecular compound together with the NSAIDs is not limited to the disaccharide, and any compound may be used, so long as it can form the intermolecular compound together with the NSAIDs. As described above, when the intermolecular compound is formed, the skin disorder induced by the NSAIDs can be suppressed. In addition, when the external preparation contains any one of maltose, sucrose and lactose, it can function as a stabilizer for the external preparation.

Examples of the present invention will be described below, but the invention is not limited thereto.

### Example 1

### Study of Intermolecular Interaction of Trehalose and NSAIDs

### 1. Test Substance

As NSAIDs, aspirin, indomethacin, ibuprofen, and diclofenac sodium were used. Aspirin, indomethacin and ibuprofen were purchased from Wako Pure Chemical Industries, Ltd., and diclofenac sodium was purchased Cayman Chemical Company. Trehalose manufactured by Hayashibara Biochemical Laboratories, Inc. and carboxymethyl cellulose·sodium (CMC·Na) manufactured by DAI-ICHI Kogyo Seiyaku Co., Ltd. were used.

### 2. Preparation of Lyophilized Product of Trehalose and NSAIDs

A 30% (w/v) solution of trehalose was prepared with purified water (milli Q grade (milli Q water)). Various NSAIDs were dissolved in ethyl alcohol (99.5%) in adequate amounts. The resulting solution was mixed with the trehalose solution, in a desired ratio, and the mixture was thoroughly stirred. After that, the mixture was dried for 48 hours or more using a lyophilizing machine (EYELA lyophilizing machine, FDU-1100 manufactured by Tokyo Rikakikai Co., Ltd.).

Specifically, the following procedures were performed.
(1) Trehalose was dissolved in milli Q water to form a 30 w/v% trehalose solution.
(2) 1.0 g of NSAIDs was dissolved in 2.0 mL of ethyl alcohol.
(3) A necessary amount of the trehalose solution was added to each of the ethyl alcohol solutions of NSAIDs. If the whole necessary amount of the trehalose solution is added to the solution of indomethacin or ibuprofen in this stage, indomethacin or ibuprofen is precipitated. For that reason, the trehalose solution was added in a maximum amount which does not cause the precipitation, and the mixture was stirred for about 10 to 20 minutes.
(4) Milli Q water was added to the mixture in an adequate amount so that the final concentration of ethyl alcohol was 10% or less (more preferably 5% or less). Precipitations of aspirin and diclofenac sodium were not almost observed, and thus the mixtures were stirred for about 10 to 20 minutes in this stage.
(5) The mixtures were dried for 48 hours or more in a lyophilizing machine (EYELA lyophilizing machine, FDU-1100 manufactured by Tokyo Rikakikai Co., Ltd.).

### Study of Intermolecular Interaction of Trehalose and NSAIDs

In order to study the intermolecular interaction of NSAIDs (indomethacin, ibuprofen, aspirin, diclofenac, piroxicam or mefenamic acid) and trehalose, measurements were performed using a differential scanning calorimetry (DSC). Measurements of trehalose alone, NSAIDs alone, a mixture of trehalose and NSAIDs, and lyophilized product of trehalose and NSAIDs were performed using the DSC. A weight ratio of trehalose and each NSAIDs is shown in Table 5 below.

### [Table 1]

**Table 1**

| NSAIDs | : | trehalose | | weight ratio |
|---|---|---|---|---|
| indomethacin | : | trehalose | = | 3 : 80 |
| ibuprofen | : | trehalose | = | 1 : 2 |
| aspirin | : | trehalose | = | 1 : 4 |
| diclofenac | : | trehalose | = | 1 : 20 |
| piroxicam | : | trehalose | = | 3 : 80 |
| mefenamic acid | : | trehalose | = | 1: 4 |

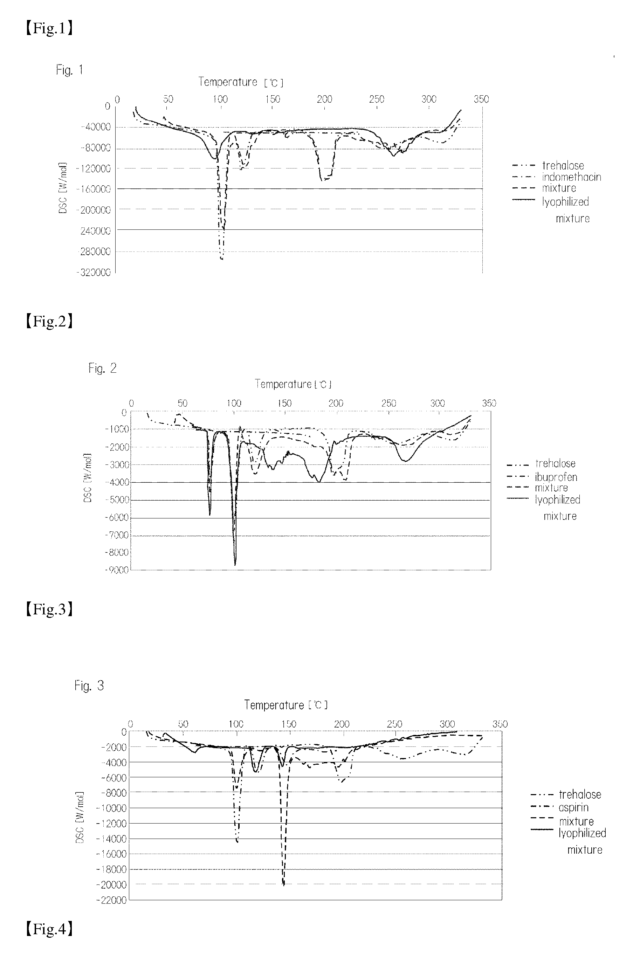

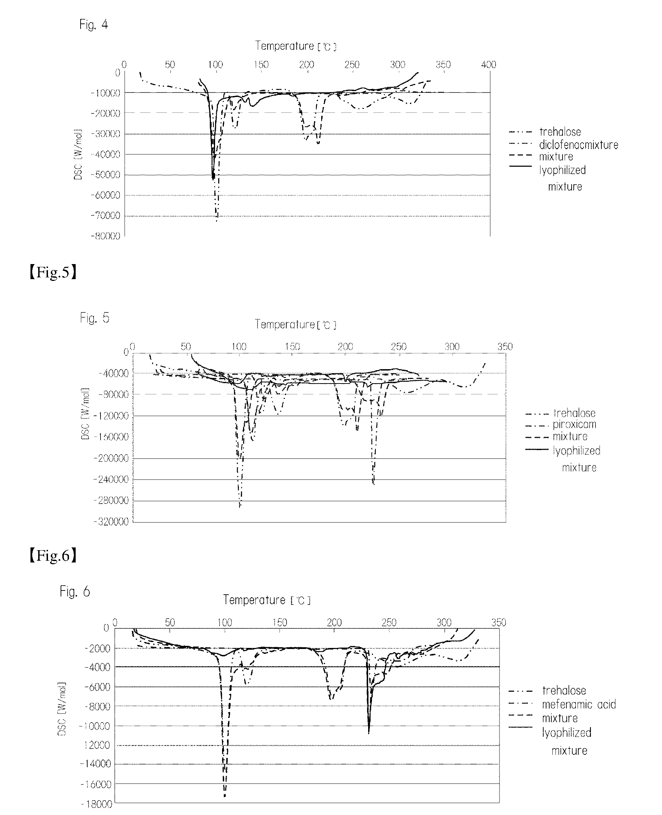

The DSC measurement results are shown in FIG. 1 to FIG. 6. FIG. 1 shows DSC results of indomethacin, FIG. 2 shows DSC results of ibuprofen, FIG. 3 shows DSC results of aspirin, FIG. 4 shows DSC results of diclofenac, FIG. 5 shows DSC results of piroxicam, and FIG. 6 shows DSC results of mefenamic acid. In FIG. 1 to FIG. 6, "mixture" shows the DSC measurement results of the mixture of trehalose and NSAIDs. In FIG. 1 to FIG. 6, "lyophilization" shows the DSC measurement results of the lyophilized product of trehalose and NSAIDs. In FIG. 1 to FIG. 6, a vertical axis shows a heat flow (W/mol) per unit mole of trehalose or NSAIDs for trehalose alone or NSAIDs alone, and a heat flow (W/mol) per unit mole of trehalose for the mixture or the lyophilized product. In FIG. 1 to FIG. 6, a horizontal axis shows a temperature (Celsius degree).

The results of FIG. 1 to FIG. 6 show that a peak of the mixture of only the NSAIDs and trehalose was close to a peak of the sum of a peak of NSAIDs alone and a peak of trehalose alone. On the other hand, in the lyophilized product, a peak derived from trehalose, which appears at 120°C, disappeared or was shifted to a side at which a temperature is lower or higher than 120°C. From these results, it was shown that there was an interaction between NSAIDs and trehalose.

In addition, from the results shown in FIG. 1, the mixture of indomethacin and trehalose apparently had a first peak at 98 to 102°C, a second peak at 190 to 210°C, and a third peak at 115 to 125°C. The first peak means the highest peak (the peak having the largest absolute value measured by DSC) on the DSC curve. The results of the mixture are almost coincided with the peaks on the DSC curve of trehalose alone. On the other hand, the lyophilized product of indomethacin and trehalose apparently had a first peak at 80 to 95°C, and a second peak at 260 to 270°C (in particular, at 264 to 266°C). In addition, the lyophilized product of indomethacin and trehalose was observed to further have a third peak at 270 to 280°C. Further, the lyophilized product of indomethacin and trehalose was observed to have no peak derived from trehalose at 190 to 210°C, which is observed in the mixture of indomethacin and trehalose. As described above, because the mixture was different from the lyophilized product in the DSC results, the intermolecular compound of indomethacin and trehalose was apparently formed by dissolving indomethacin together with trehalose and then lyophilizing the solution.

From the results shown in FIG. 2, the mixture of ibuprofen and trehalose apparently had a first peak at 98 to 102°C, a second peak at 70 to 80°C, a third peak at 190 to 210°C, and a fourth peak at 115 to 125°C. The results of the mixture are almost coincided with the peaks on the DSC curve of trehalose alone and the peaks on the DSC curve of the ibuprofen alone. On the other hand, the lyophilized product of ibuprofen and trehalose apparently had a first peak at 98 to 102°C, a second peak at 70 to 80°C, a third peak at 175 to 190°C, and a fourth peak at 130 to 145°C. The mixture of ibuprofen and trehalose apparently had no peaks at 175 to 190°C or at 130 to 145°C on the DES curve, which peaks appeared on the DSC curve of the lyophilized product of ibuprofen and trehalose. As described above, because the mixture was different from the lyophilized product in the DSC results, the intermolecular compound of the ibuprofen and trehalose was apparently formed by dissolving ibuprofen together with trehalose and then lyophilizing the solution.

From the results shown in FIG. 3, the mixture of aspirin and trehalose apparently had a first peak at 145 to 150°C, and a second peak at 98 to 102°C. On the other hand, the lyophilized product of aspirin and trehalose apparently had a first peak at 110 to 120°C (in particular, at 118 to 119°C), and a second peak at 135 to 145°C. The lyophilized product of aspirin and trehalose was observed to have no peak derived from aspirin at 98 to 102°C. In addition, the lyophilized product of aspirin and trehalose was also observed to have no peak derived from aspirin at 190 to 220°C. On the other hand, the mixture of aspirin and trehalose was observed to have no peak having high intensity at 110 to 120°C. As described above, because the mixture was different from the lyophilized product in the DSC results, the intermolecular compound of aspirin and trehalose was apparently formed by dissolving aspirin together with trehalose and then lyophilizing the solution.

From the results shown in FIG. 4, the mixture of diclofenac sodium and trehalose apparently had a first peak at 95 to 110°C, and a second peak at 190 to 220°C. On the other hand, the lyophilized product of diclofenac sodium and trehalose apparently had a first peak at 90 to 100°C, and a second peak at 135 to 145°C. The mixture of diclofenac sodium and trehalose was observed to have no peak at 135 to 145°C. On the other hand, the lyophilized product of diclofenac sodium and trehalose was observed to have no peak at 190 to 220°C. Although the mixture of diclofenac sodium and trehalose was observed to have a peak at 110 to 120°C (in particular, at around 119°C), the lyophilized product of diclofenac sodium and trehalose was observed to have no peak at 110 to 120°C. As described above, because the mixture was different from the lyophilized product, the intermolecular compound of diclofenac and trehalose was apparently formed by dissolving diclofenac together with trehalose and then lyophilizing the solution.

From the results shown in FIG. 5, the mixture of mefenamic acid and trehalose apparently had a first peak at 98 to 102°C, a second peak at 225 to 235°C, a third peak at 190 to 210°C. In addition, the mixture of mefenamic acid and trehalose also had a fourth peak at 115 to 125°C. On the other hand, lyophilized product of mefenamic acid and trehalose apparently had a first peak at 225 to 235°C, and a second peak at 90 to 110°C. In addition, the lyophilized product of mefenamic acid and trehalose also had a peak at 180 to 190°C (at 185 to 187°C). Further, the lyophilized product of mefenamic acid and trehalose also had a peak at 250 to 265°C (255 to 260°). The lyophilized product of mefenamic acid and trehalose was observed to have no peak at 115 to 125°C. The absolute values (the absolute values of the value measured by DSC) of the tops of the first peak and the second peak of the lyophilized product are larger than the absolute values of the tops of the peaks at 225 to 235°C and 90 to 110°C on the DSC curve of mefenamic acid alone. As described above, because the mixture was different from the lyophilized product in the DSC results, and mefenamic acid alone was different from the lyophilized product in the DSC results, the intermolecular compound of mefenamic acid and trehalose was apparently formed by dissolving mefenamic acid together with trehalose and then lyophilizing the solution.

From the results shown in FIG. 6, the mixture of piroxicam and trehalose apparently had a first peak at 98 to 102°C, a second peak at 225 to 235°C, and a third peak at 205 to 215°C. The mixture of piroxicam and trehalose also had a peak at 120 to 130°C. On the other hand, the lyophilized product of piroxicam and trehalose apparently had a first peak at 90 to 105°C, and a second peak at 195 to 205°C (in particular, 198 to 200°C). The lyophilized product of piroxicam and trehalose had no peak at 205° to 215°C. At least, the lyophilized product of piroxicam and trehalose had main peaks at 190 to 220°C and 195 to 205°C (in particular, 198 to 200°C).
The absolute values of the tops of the first peak and the second peak of the lyophilized product are larger than the absolute values of the tops of the peaks at 90 to 105°C and 195 to 205°C on the DSC curve of piroxicam alone. As described above, because the mixture was different from the lyophilized product in the DSC results, and piroxicam alone was different from the lyophilized product in the DSC results, the intermolecular compound of piroxicam and trehalose was apparently formed by dissolving piroxicam together with trehalose and then lyophilize the solution.

### Example 2

### Influence of NSAIDs-Containing Ointment Drug Formulation on Cell Viability

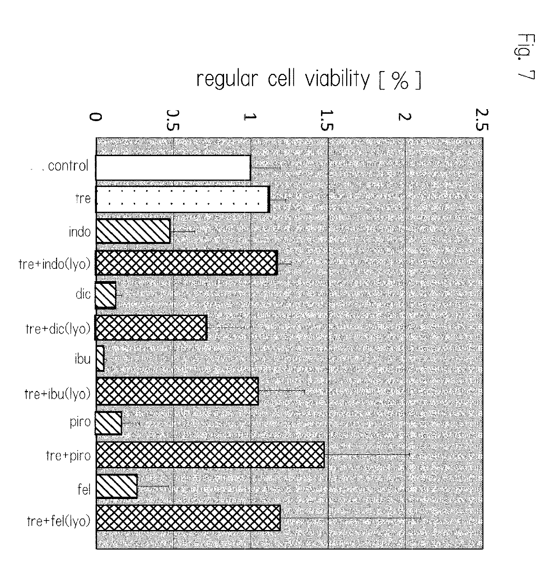

Influence of an NSAIDs-containing ointment on cell viability was studied using TEST SKIN manufactured by TOYOBO Co., Ltd, which was a human skin-reconstitution model. Intermolecular compound preparations containing NSAIDs and trehalose shown in Table 2 were obtained by lyophilizing mixed solutions containing NSAIDs and trehalose. Each NSAIDs-containing ointment was obtained by mixing the intermolecular compound preparation and a hydrophilic ointment so that a final concentration (w/v%) of the NSAIDs was a concentration shown in the right column of Table 2 below. A surface of the TEST SKIN, which had been sealed with a silicon ring, was coated with 100 mg of each NSAIDs-containing ointment. After that, TEST SKIN was cultivated at 37°C for 20 hours in an incubator. The ointment, which had been coated on the surface, was washed off so that the tissue was not damaged. After the cultivation was performed in an assay culture medium in which MTT was dissolved for further 3 hours, MTT color development was confirmed, and the cultivated skin was cut with a punch. The cut tissue slice was immersed in 300 µL of 0.04 M of a solution of hydrochloric acid-isopropyl alcohol (HCI-IPA), and blue formazan was extracted for 16 hours at room temperature under shading. The extracted solution was measured using a spectrophotometer. A cell viability was calculated from an absorbance at 570 nm. The results are shown in FIG. 7. In FIG. 7, the sign "tre" indicates trehalose; "indo" indicates indomethacin; "(lyo)" indicates a lyophilized product; "dic" indicates diclofenac; "ibu" indicates ibuprofen; "piro" indicates piroxicam; and "fel" indicates felbinac. In FIG. 7, "control" indicates a case in which cultivated skin alone, to which an ointment was not added, was cultivated for 20 hours, and then the MTT assay was performed in the same manner as above, which corresponds to a control experiment. "Control" in FIG. 7, thus, is the standard of cell viability 100%.

### [Table 2]

**Table 2**

| NSAIDs | : | trehalose | mixing ratio | final concentration |
|---|---|---|---|---|
| trehalose only | : | | - | 10% |
| indomethacin | : | trehalose | 3:80 | 1.0% |
| ibuprofen | : | trehalose | 1 : 2 | 1.0% |
| diclofenac | : | trehalose | 1 : 20 | 5.0% |
| piroxicam | : | trehalose | 3 : 80 | 0.5% |
| felbinac | : | trehalose | 1 : 5 | 3.0% |

The results of FIG. 7 show apparently that, for all NSAIDs, the cell viabilities of products obtained by intermolecular interaction of NSAIDs with trehalose are higher than those of NSAIDs alone. It was shown, therefore, that cell dysfunctions induced by NSAIDs could be suppressed by the intermolecular interaction of trehalose with NSAIDs.

### Example 3

### Cytotoxicity Test of NSAIDs on Cultivated Skin

Experiments (cytotoxicity test) for testing cytotoxicities of the mixture of NSAIDs and trehalose, and the intermolecular compound of NSAIDs and trehalose were performed.

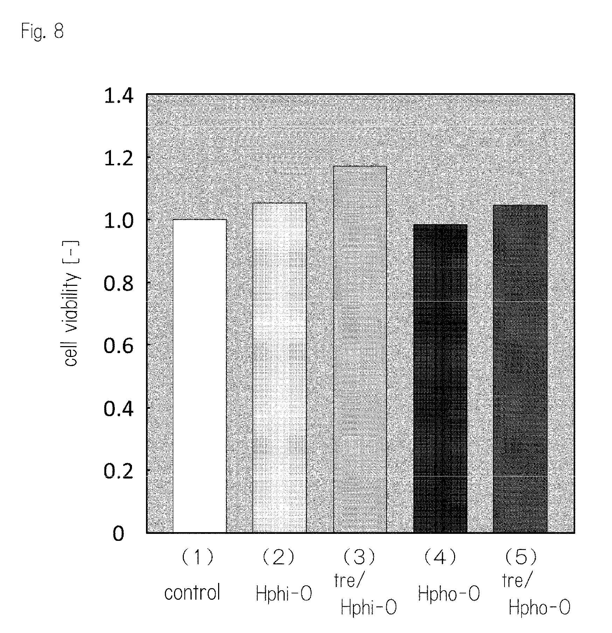

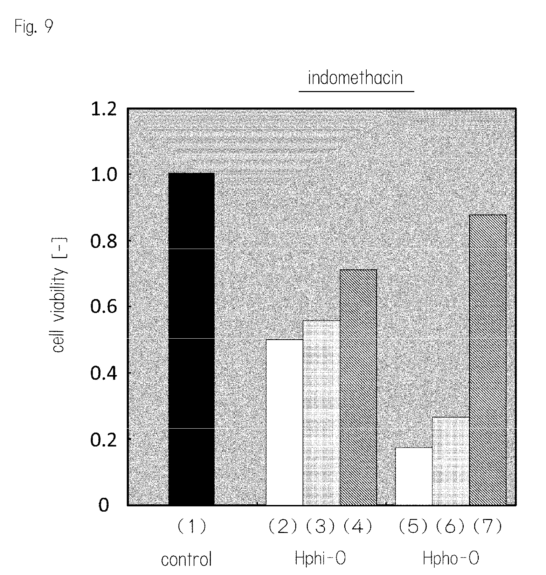

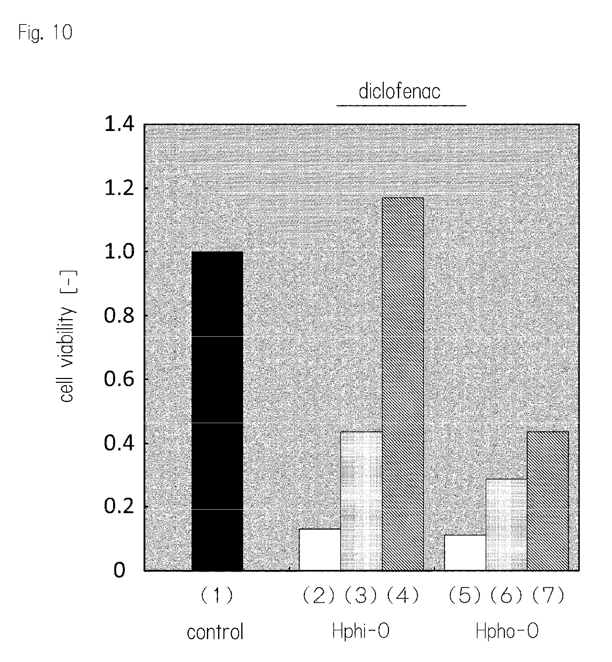

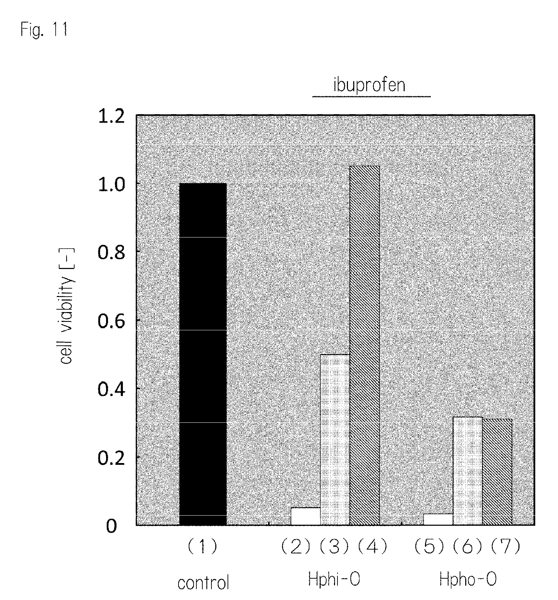

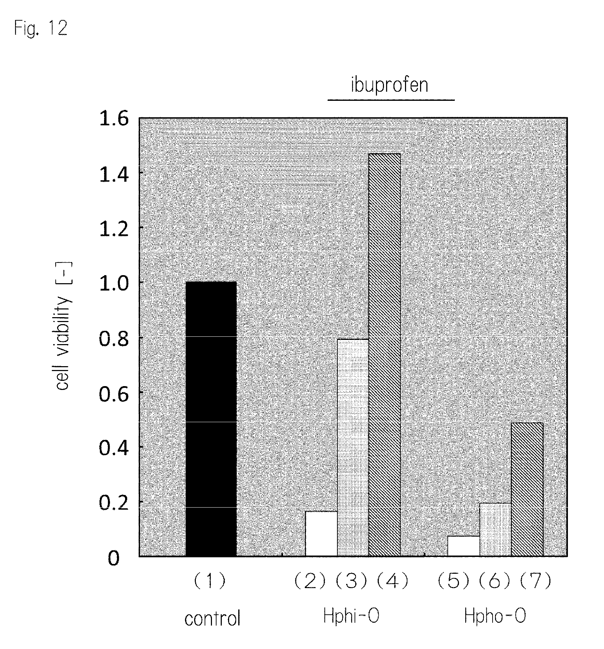

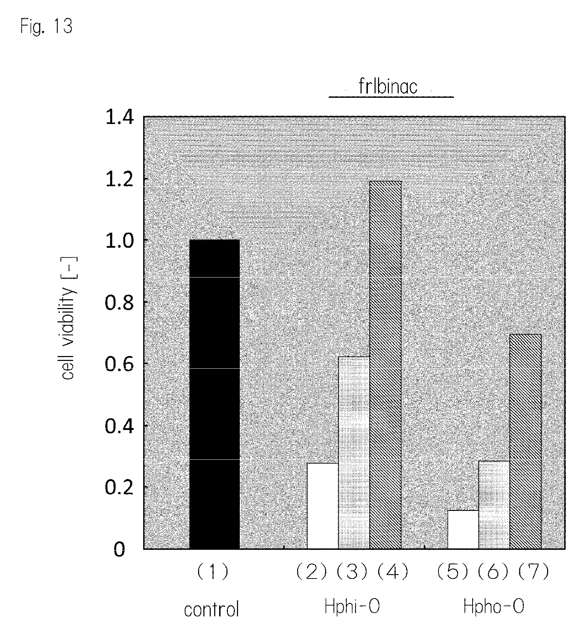

FIG. 8 to FIG. 13 show cell viabilities, which are results of cytotoxicity tests in Example 3, performed in the same manner as in Example 2. In each FIG, Hphi-O indicates a hydrophilic ointment, and Hpho-O indicates a hydrophobic ointment. FIG. 8 shows cell viabilities of control experiments in which NSAIDs was not contained. Specifically, FIG. 8 shows cytotoxicity test results of, from the left side, (1) a control, (2) a hydrophilic ointment, (3) a trehalose hydrophilic ointment, (4) a hydrophobic ointment, and (5) a trehalose hydrophobic ointment are shown. FIG. 9 to FIG. 13 show the results of cytotoxicity tests in which NSAIDs was contained. Specifically, FIG. 9 to FIG. 13 shows the results of, from the left side, (1) a control, (2) an NSAIDs hydrophilic ointment, (3) a hydrophilic ointment of a mixture of NSAIDs and trehalose, (4) a hydrophilic ointment of lyophilized intermolecular compound of NSAIDs and trehalose, (5) an NSAIDs hydrophobic ointment, (6)a hydrophobic ointment of a mixture of NSAIDs and trehalose, and (7) a hydrophobic ointment of a lyophilized intermolecular compound of NSAIDs and trehalose. Here, "control" in FIG. 9 to FIG. 13 is one cultivated with a cultivated skin alone. "Mixture" is one obtained by separately grinding trehalose and NSAIDs in mortars, and then adding trehalose and NSAIDs in pre-determined amount to an ointment. "Intermolecular compound: lyopholization" is one obtained by lyophilizing a mixture of trehalose and NSAIDs to obtain a compound and adding the compound to an ointment so that the NSAIDs is added in a pre-determined amount. Indomethacin (FIG. 9), diclofenac (FIG. 10), ibuprofen (FIG. 11), piroxicam (FIG. 12), and felbinac (FIG. 13) were used as the NSAIDs.

FIG. 8 shows that less cytotoxicity was observed in the control experiment containing no NDAIDs. On the other hand, From FIG. 9 to FIG. 13, it was found that in the case containing NSAIDs, the intermolecular compound of NSAIDs and trehalose could considerably suppress the cytotoxicity derived from NSAIDs, compared to the mixture of NSAIDs and trehalose. In particular, the suppression of the cytotoxicity by the intermolecular compound was considerably shown in the case using the hydrophobic ointment. It was shown, therefore, that the external preparation of the invention was more effectively used in the case using as the hydrophobic ointment.

### Example 4

In Example 4, the same experiment as in Example 2 was performed using maltose instead of trehalose. That is, an intermolecular compound was formed from maltose and NSAIDs.
Specifically, a lyophilized product of diclofenac and maltose (Dic-Mal) in a weight ratio of 1:20 was added to a DMEM medium (manufactured by Sigma Corporation) so that a final concentration of diclofenac was 1 mM, and it was completely dissolved in the medium. The medium was added to epithelial cells, Ca 9-22 cells, which was cultivated for 16 hours. After that, cell viability was measured. The cell viability was measured using a LIVE/DEAD viability toxicity kit of Molecular Probes (registered trademark) manufactured by Invitrogen Com.

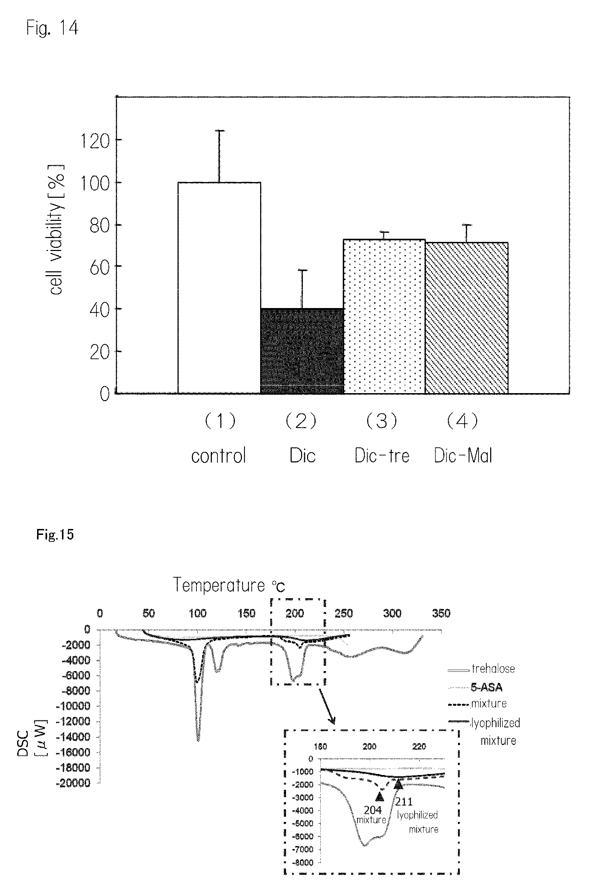

FIG. 14 shows measurement results of cell viability when diclofenac was used as NSAIDs. FIG. 14 also shows the results of a case using no diclofenac (control), a case using diclofenac alone but no intermolecular compound (Dic), and a case using trehalose (Tre). In the lyophilized product of diclofenac and trehalose (Dic-Tre), the weight ratio of diclofenac and trehalose was 1:20.

As seen from FIG. 14, an effect of protecting cells (that is, the effect of suppressing the cell dysfunction), which was equal to or higher than that of trehalose, could be exhibited when NSAIDs formed the intermolecular compound with maltose. Here, both maltose and trehalose are the same disaccharides. It is suggested, therefore, that the intermolecular compound formed from any disaccharide and NSAIDs can suppress the cell dysfunctions induced by NSAIDs. The disaccharide may be exemplified by sucrose and lactose in addition to maltose and trehalose.

### Example 5

### DSC Measurement of 5-Aminosalicylic Acid (5-ASA)

DSC experiments of 5-ASA were performed in the same manner as in Example 1. FIG. 15 shows charts, replacing for a drawing, which show DSC results of trehalose alone, 5-ASA alone, a mixture of 5-ASA and trehalose, and lyophilized mixture of 5-ASA and trehalose.

As shown in FIG. 15, the mixture of 5-ASA and trehalose had a first peak at 95 to 105°C, and a second peak at 200 to 205°C. The lyophilized product of 5-ASA and trehalose had a peak at 207 to 215°C. On the other hand, the lyophilized product of 5-ASA and trehalose was observed to have no peak at 95 to 105°C.

### Example 6

### DSC Measurement of Ketoprofen

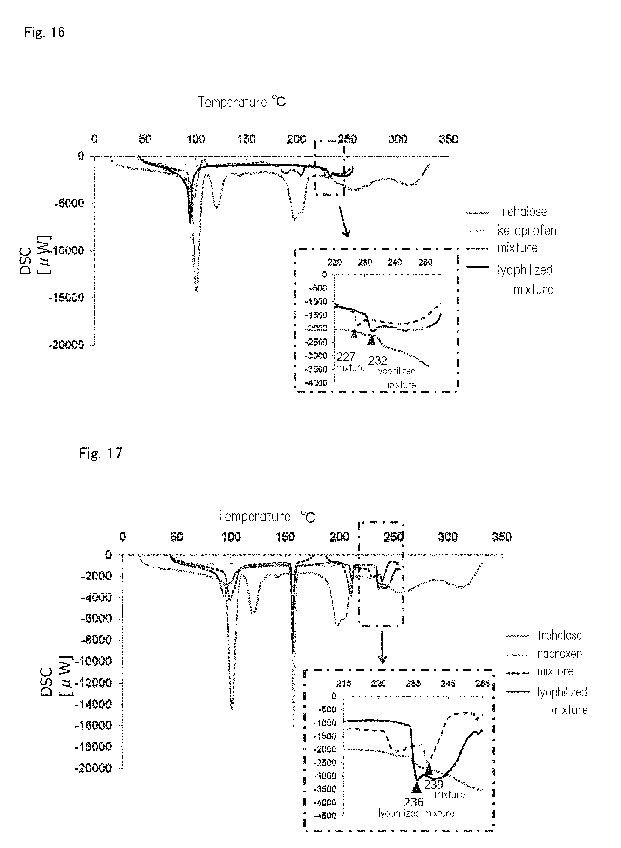

DSC experiments of ketoprofen were performed in the same manner as in Example 1. FIG. 16 shows charts, replacing for a drawing, which show DSC results of trehalose alone, ketoprofen alone, a mixture of ketoprofen and trehalose, and a lyophilized mixture of ketoprofen and trehalose.

As shown in FIG. 16, the lyophilized product of ketoprofen and trehalose had a DSC curve, obtained by the differential scanning calorimetry (DSC) measurement, in which peaks appeared at 90 to 95°C and 230 to 235°C, but no peak appeared at 180 to 220°C.

### Example 7

### DSC Measurement of Naproxen

DSC experiments of naproxen were performed in the same manner as in Example 1. FIG. 17 shows charts, replacing for a drawing, which show DSC results of trehalose alone, naproxen alone, a mixture of naproxen and trehalose, and lyophilized mixture of naproxen and trehalose.

As shown in FIG. 17, the lyophilized product of naproxen and trehalose had a DSC curve, obtained by the differential scanning calorimetry (DSC) measurement, in which peaks appeared at 90 to 95°C and 234 to 237°C, but no peak appeared at 225 to 233°C.

### Example 8

In Example 8, it was the presence of an intermolecular compound was confirmed by comparing the FT-IR spectra of an intermolecular compound (lyophilized product) of NSAIDs and trehalose with the FT-IR spectra of a mixture of NSAIDs and trehalose.

Data of intermolecular compounds of NSAIDs and trehalose were divided into salicylic acid NSAIDs, arylacetic acid NSAIDs, propionic acid NSAIDs, oxicam NSAIDs, and fenamic acid NSAIDs, and FT-IR data thereof were measured.

Samples to be measured by FT-IR (KBr method) contained NSAIDs and trehalose in a molar ratio of 1:1. This is because if the amount of trehalose is large, the spectra of the intermolecular compound are hidden, since FT-IR is a mycloanalysis. As for aspirin, measurement was performed at the molar ratio of aspirin:trehalose being 2:1.

FT-IR measurement was performed as follows: KBr was taken in an adequate amount with a drug spoon, and it was ground in a mortar. After KBr was uniformly and finely ground, a sample to be measured was added to KBr in an adequate amount with a drug spoon, and the mixture was ground. After KBr and the sample were uniformly mixed, the mixture was put in a mold, and a pressure (20 kPa) was applied thereto to produce a film-like plate of KBr. The plate was set in an FT-IR apparatus (an FT-IR 615 JASCO), and measurement was performed at 650 to 4000 cm⁻¹.

Using aspirin or 5-ASA as a typical salicylic acid NSAIDs, in order to determine whether the intermolecular compound thereof was produced or not, FT-IR spectra were measured.

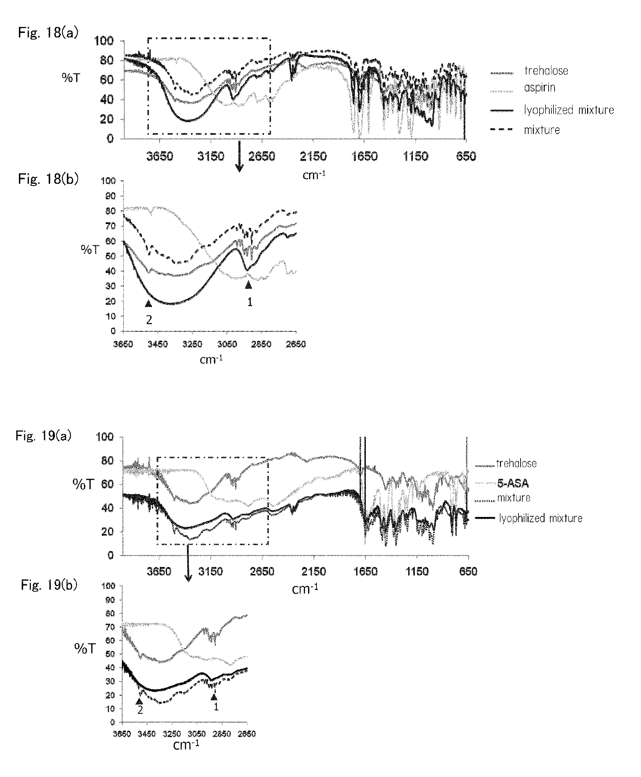

FIG. 18 shows FT-IR spectra of aspirin alone, trehalose, a mixture of aspirin and trehalose, and lyophilized mixture of aspirin and trehalose. In the figure, the numeral 1 indicates a top of peak of CH group. The mixture had a top of peak at 2907 cm⁻¹. The lyophilized product had a top of peak at 2931 cm⁻¹. In the figure, the numeral 2 indicate a top of peak of OH group. The mixture had a top of peak at 3500 cm⁻¹. The lyophilized product had a broad peak.

The measurement was performed for trehalose, aspirin, the mixture or the lyophilized product. As a result, when the spectral shape of the mixture was compared with that of the lyophilized product, changes were observed at around OH group and around CH group. In particular, the spectrum of the lyophilized product became broad at both OH group and CH group, and the top of peak of CH group was changed. From these results, it could be considered that there was an interaction between trehalose and aspirin.

FIG. 19 shows FT-IR spectra of 5-ASA alone, trehalose, a mixture of 5-ASA and trehalose, and lyophilized mixture of 5-ASA and trehalose. In the figure, the numeral 1 indicates a top of peak of CH group. The mixture had a top of peak at around 2907 cm⁻¹. The lyophilized product had a top of peak at 2927 cm⁻¹. In the figure, the numeral 2 indicates a top of peak of OH group. The mixture had a top of peak at around 3500 cm⁻¹. The lyophilized product had a broad peak. The measurement was performed for trehalose, 5-ASA, the mixture or the lyophilized product. As a result, when the mixture was compared with the lyophilized product, spectral shapes of OH group and CH group were changed. The peak of OH group became broad for the lyophilized product, and the top of peak of CH group was also changed. From these results, it could be considered that there was an interaction between trehalose and 5-ASA.

Using diclofenac or indomethacin as a typical aryl acid NSAIDs, in order to determine whether the intermolecular compound thereof was formed or not, FT-IR spectra were measured.

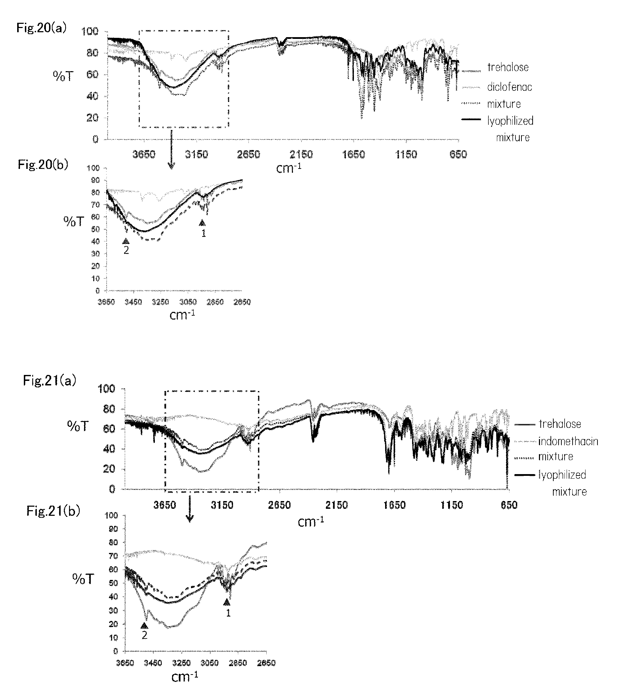

FIG. 20 shows FT-IR spectra of diclofenac alone, trehalose, a mixture of diclofenac and trehalose, and lyophilized mixture of diclofenac and trehalose. In the figure, the numeral 1 indicates a top of peak of CH group. The mixture had a top of peak at 2907 cm⁻¹. The lyophilized product had a top of peak of CH group at around 2933 cm⁻¹. In the figure, the numeral 2 indicate a top of peak of OH group. The mixture had a top of peak at around 3500 cm⁻¹. The lyophilized product had a broad peak. The measurement was performed for trehalose, diclofenac, the mixture or the lyophilized product. As a result, when the mixture was compared with the lyophilized product, the spectral shapes of OH group and CH group were changed. The spectral shape of OH group and the position of the top of peak of CH group were changed. From these results, it could be considered that there was an interaction between trehalose and diclofenac.

FIG. 21 shows FT-IR spectra of indomethacin alone, trehalose, a mixture of indomethacin and trehalose, and lyophilized mixture of indomethacin and trehalose. In the figure, the numeral 1 indicates a top of peak of CH group. The mixture had a top of peak at 2907 cm⁻¹. The lyophilized product had a top of peak of CH group at around 2927 cm⁻¹. In the figure, the numeral 2 indicates a top of peak of OH group. The mixture had a top of peak at around 3500 cm⁻¹. The lyophilized product had a broad peak. The measurement was performed for trehalose, indomethacin, the mixture or the lyophilized product. As a result, when the mixture was compared with the lyophilized product, the spectral shapes of OH group and CH group were changed. The spectral shape of OH group and the position of the top of peak of CH group were changed for the lyophilized product. It could be considered that there was an interaction between trehalose and indomethacin.

Using ibuprofen, ketoprofen or naproxen as a typical propionic acid NSAIDs, in order to determine whether the intermolecular compound thereof was formed or not, FT-IR spectra were measured.

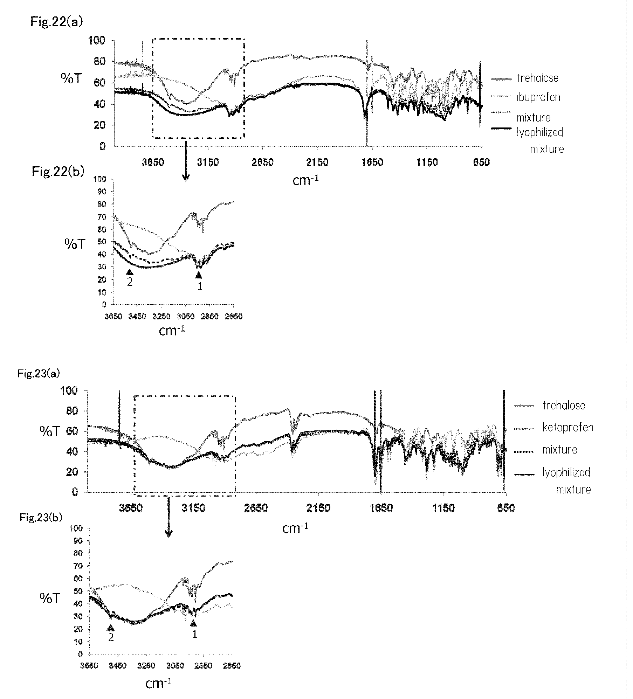

FIG. 22 shows FT-IR spectra of ibuprofen alone, trehalose, a mixture of ibuprofen and trehalose, and a lyophilized mixture of ibuprofen and trehalose. In the figure, the numeral 1 indicates a top of peak of CH group. The mixture had a top of peak at 2907 cm⁻¹. The lyophilized product had a top of peak of CH group at around 2922 cm⁻¹. In the figure, the numeral 2 indicates a top of peak of OH group. The mixture had a top of peak at around 3500 cm⁻¹. The lyophilized product had a broad peak. The measurement was performed for trehalose, ibuprofen, the mixture and the lyophilized product. As a result, when the mixture was compared with the lyophilized product, the spectral shapes of OH group and CH group were changed. The peaks of OH group and CH group became broad for the lyophilized product, and the position of the top of peak of CH group was changed. From these results, it could be considered that there was an interaction between trehalose and ibuprofen.

FIG. 23 shows FT-IR spectra of ketoprofen alone, trehalose, a mixture of ketoprofen and trehalosem, and lyophilized mixture of ketoprofen and trehalose. In the figure, the numeral 1 indicates a top of peak of CH group. The mixture had a top of peak at 2907 cm⁻¹. The lyophilized product had a top of peak of CH group at around 2922 cm⁻¹. In the figure, the numeral 2 indicates a top of peak of OH group. The mixture had a top of peak at around 3500 cm⁻¹. The lyophilized product had a broad peak. The measurement was performed for trehalose, ketoprofen, the mixture and the lyophilized product. As a result, when the mixture was compared with the lyophilized product, the spectral shapes of OH group and CH group were changed. The peaks of OH group and CH group became broad for the lyophilized product, and the position of the top of peak of CH group was changed. From these results, it could be considered that there was an interaction between trehalose and ketoprofen.

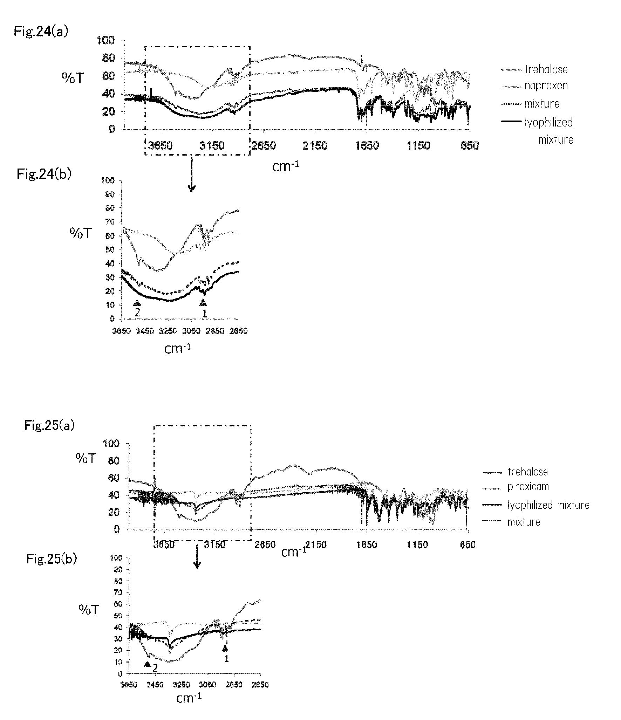

FIG. 24 shows FT-IR spectra of naproxen alone, trehalose, a mixture of naproxen and trehalose, and a lyophilized mixture of naproxen and trehalose. In the figure, the numeral 1 indicates a top of peak of CH group. The mixture had a top of peak at 2907 cm⁻¹. The lyophilized product had a top of peak at around 2938 cm⁻¹. In the figure, the numeral 2 indicates a top of peak of OH group. The mixture had a top of peak at around 3500 cm⁻¹. The lyophilized product had a broad peak. The measurement was performed for trehalose, naproxen, the mixture and the lyophilized product. As a result, when the mixture was compared with the lyophilized product, the spectral shapes of OH group and CH group were changed. The peaks of OH group and CH group became broad for the lyophilized product, and the position of the top of peak of CH group was changed. From these results, it could be considered that there was an interaction between trehalose and naproxen.

Using piroxicam as a typical oxicam NSAIDs, in order to determine whether the intermolecular compound thereof was formed or not, FT-IR spectra were measured.

FIG. 25 shows FT-IR spectra of piroxicam alone, trehalose, a mixture of piroxicam and trehalose, and a lyophilized mixture of piroxicam and trehalose. In the figure, the numeral 1 indicates a top of peak of CH group. The mixture had a top of peak at 2907 cm⁻¹. The lyophilized product had top of peak of CH group at around 2932 cm⁻¹. In the figure, the numeral 2 indicates a top of peak of OH group. The mixture had a top of peak at around 3500 cm⁻¹. The lyophilized product had a broad peak. The measurement was performed for trehalose, piroxicam, the mixture and the lyophilized product. As a result, when the mixture was compared with the lyophilized product, the spectral shape of OH group and CH group were changed. The peak of OH group became broad, and the position of the top of peak of CH group was changed for the lyophilized product. From these results, it could be considered that there was an interaction between trehalose and piroxicam.

Using mefenamic acid as a typical fenamic NSAIDs, in order to determine whether the intermolecular compound thereof was formed or not, FT-IR spectra were measured.

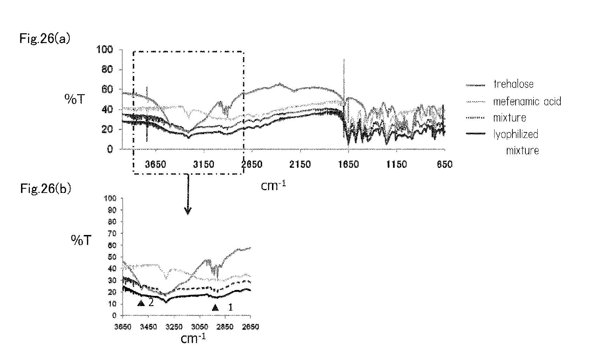

FIG. 26 shows FT-IR spectra of mefenamic acid alone, trehalose, a mixture of mefenamic acid and trehalose, and a lyophilized mixture of mefenamic acid and trehalose. In the figure, the numeral 1 indicates a top of peak of CH group. Change in the top of peak of CH group was not observed in the mixture and the lyophilized product. In the figure, the numeral 2 indicates a top of peak of OH group. The mixture had a top of peak at around 3500 cm⁻¹. The peak became broad for the lyophilized product. The measurement was performed for trehalose, mefenamic acid, the mixture and the lyophilized product. As a result, when the mixture was compared with the lyophilized product, the spectral shapes of OH group and CH group were changed. Although the wave number of the top of peak of CH group was not changed, the shape of the spectrum became broad. From these results, it could be considered that there was an interaction between trehalose and mefenamic acid.

### Industrial Applicability

The present invention can be used in the pharmaceutical industry.

## Claims

1. An External Preparation which contains an intermolecular compound obtained from a nonsteroidal anti-inflammatory drug (NSAIDs) and a disaccharide, thereby the External Preparation suppresses skin disorders induced by NSAIDs.

2. The External Preparation in accordance with claim 1,
wherein the intermolecular compound is obtained by drying a mixed solution containing the NSAIDs and the disaccharide.

3. The External Preparation in accordance with claim 1,
wherein the disaccharide is trehalose, and
wherein the intermolecular compound contains the NSAIDs and the trehalose in a weight ratio of 10:1 to 1:50.

4. The External Preparation in accordance with claim 1,
wherein the NSAIDs is acidic NSAIDs.

5. The External Preparation in accordance with claim 1,
wherein the NSAIDs contains any one or two or more of indomethacin, ibuprofen, aspirin, diclofenac sodium, mefenamic acid, piroxicam, loxoprofen, ketoprofen, flurbiprofen, glycol salicylate, glycyrrhetinic acid, Loxonin, suprofen, bufexamac, ufenamate, dimethylisopropylazulene , 5-aminosalicylic acid and naproxen.

6. The External Preparation in accordance with claim 1,
wherein the NSAIDs is felbinac.

7. The External Preparation in accordance with claim 1,
wherein the external preparation further includes an oleaginous base.

8. The External Preparation in accordance with claim 1,
wherein the external preparation further contains a cosolvent.

9. The External Preparation in accordance with claim 1,
wherein the external preparation further contains a local anesthetic.

10. The External Preparation in accordance with claim 1,
wherein the external preparation further contains any one kind or two kinds or more of lidocaine, tetracaine, procaine, dibucaine, benzocaine, bupivacaine, mepivacaine, ethyl aminobenzoate, diethylaminoethyl para-butylaminobenzoate, meprylcaine, oxypolyethoxydodecane, and scopolia extract and a salt thereof.

11. The External Preparation in accordance with claim 1,
wherein the NSAIDs is indomethacin, and
wherein a DSC curve of the intermolecular compound obtained by the method of differential scanning calorimetry (DSC) has a first peak and a second peak at 80 to 95 °C and 260 to 270 °C, respectively.

12. The External Preparation in accordance with claim 1,
wherein the NSAIDs is ibuprofen, and
wherein a DSC curve of the intermolecular compound obtained by the method of differential scanning calorimetry (DSC) has a third peak and a fourth peak at 175 to 190 °C and 130 to 145 °C, respectively.

13. The External Preparation in accordance with claim 1,
wherein the NSAIDs is aspirin, and
wherein a DSC curve of the intermolecular compound obtained by the method of differential scanning calorimetry (DSC) has a first peak and a second peak at 110 to 120 °C and 135 to 145 °C, respectively.

14. The External Preparation in accordance with claim 1,
wherein the NSAIDs is diclofenac sodium, and
wherein a DSC curve of the intermolecular compound obtained by the method of differential scanning calorimetry (DSC) has a first peak and a second peak at 90 to 100 °C and 130 to 145 °C, respectively.

15. The External Preparation in accordance with claim 1,
wherein the NSAIDs is mefenamic acid, and
wherein a DSC curve of the intermolecular compound obtained by the method of differential scanning calorimetry (DSC) has a first peak and a second peak at 225 to 235 °C and 90 to 110 °C, respectively, and other peaks at 180 to 190°C and 250 to 265°C.

16. The External Preparation in accordance with claim 1,
wherein the NSAIDs is piroxicam, and
wherein a DSC curve of the intermolecular compound obtained by the method of differential scanning calorimetry (DSC) has a first peak and a second peak at 90 to 105 °C and 195 to 205 °C, respectively, and the second peak at 195 to 205 °C is a main peak in a range of 190 to 220°C.

17. The External Preparation in accordance with claim 1,
wherein the NSAIDs is 5-aminosalicylic acid, and
wherein a DSC curve of the intermolecular compound obtained by the method of differential scanning calorimetry (DSC) has a peak at 207 to 215 °C, but no peak at 95 to 105 °C.

18. The External Preparation in accordance with claim 1,
wherein the NSAIDs is ketoprofen, and
wherein a DSC curve of the intermolecular compound obtained by the method of differential scanning calorimetry (DSC) has peaks at 90 to 95 °C and 230 to 235 °C, but no peak at 180 to 220 °C.

19. The External Preparation in accordance with claim 1,
wherein the NSAIDs is naproxen, and
wherein a DSC curve of the intermolecular compound obtained by the method of differential scanning calorimetry (DSC) has peaks at 90 to 95 °C and 234 to 237 °C, but no peak at 225 to 233 °C.

20. The External Preparation in accordance with claim 1,
wherein the disaccharide is maltose.

21. The External Preparation in accordance with claim 1,
wherein the disaccharide contains any one or more of trehalose,. maltose, sucrose and lactose.

22. A method for producing an External Preparation comprising steps of:
preparing a mixed solution containing a nonsteroidal anti-inflammatory drug (NSAIDs) and a disaccharide; and
drying the mixed solution;
therby the NSAIDs and the disaccharide form an intermolecular compound to suppress the skin disorders induced by NSAIDs.

23. A method for producing an External Preparation comprising steps of:
preparing a mixed solution containing a nonsteroidal anti-inflammatory drug (NSAIDs) and a disaccharide;
drying the mixed solution to obtain a dried mixture; and
mixing the dried mixture, a base and a cosolvent;
therby the External Preparation is produced as a mixture of an intermolecular compound which is obtained at the step of drying the mixed solution, the base and the cosolvent.

24. The method in accordance with claim 22 or 23,
wherin the disaccharide contains any one or more of trehalose,. maltose, sucrose and lactose.
